Europäisches Patentamt

European Patent Office

Office européen des brevets

(19)

(11) Publication number: **0 080 108**
**B1**

# EUROPEAN PATENT SPECIFICATION

(45) Date of publication of patent specification: **08.10.86**

(21) Application number: **82110330.6**

(22) Date of filing: **09.11.82**

(51) Int. Cl.⁴: **G 01 N 33/531,**
G 01 N 33/543, C 12 Q 1/00

(54) **Diagnostic reagent and use thereof.**

(30) Priority: **19.11.81 US 323003**
**29.03.82 US 362718**

(43) Date of publication of application:
**01.06.83 Bulletin 83/22**

(45) Publication of the grant of the patent:
**08.10.86 Bulletin 86/41**

(84) Designated Contracting States:
**BE DE FR GB NL SE**

(56) References cited:
**EP-A-0 019 277**
**EP-A-0 019 504**
**US-A-4 172 117**
**US-A-4 210 622**
**US-A-4 241 175**

(73) Proprietor: **New York Blood Center, Inc.**
**310 East 67 Street**
**New York, New York 10021 (US)**

(72) Inventor: **Neurath, Robert A.**
**230 East 79th Street**
**New York, N.Y. 10021 (US)**

(74) Representative: **Reitzner, Bruno, Dr. et al**
**Patentanwälte Dipl.-Ing. R. Splanemann Dr. B.**
**Reitzner Tal 13**
**D-8000 München 2 (DE)**

Courier Press, Leamington Spa, England.

## Description

This invention relates to a diagnostic reagent which is suitable both for the detection of an antigen, and for the detection of an antibody in a specimen, by radioimmuno assay or by enzyme labeled immunoassay, said reagents comprising a substrate having bound thereon an antibody and an antigen.

Radioimmunoassay techniques for biochemical and immunological studies and for clinical research and diagnosis have become an invaluable tool. However, their applicability has been confined to reasonably well characterized antigens which can be purified and used for the preparation of antisera serving as a source for isolation of immunochemically purified antibodies. Although $^{125}$ I labeled staphylococcal protein A has been suggested as a general radioactive reagent for radioimmunoassay, it cannot be used for sandwich type tests with an antibody-coated solid phase. If neither antigen nor the corresponding antibody are available in relatively purified form, it becomes difficult to prepare radiolabeled reagents for radioimmunoassay (RIA) suitable for the detection of nanogram quantities of antigens.

It therefore became desirable to provide a process for the detection of and the quantitative measurement of antigens, which process could be used for those antibodies and antigens whose purfication into relatively purified form was not heretofore known. More especially, it became desirable to provide a process by which nanogram quantities of antigens could be detected, which process did not rely upon the purification of antibodies and antigens as source material for the test. Still more especially, it became desirable to provide a process by which one could use a universal reagent for the detection of the presence of a wide variety of different types of antigens.

EP—A—0 019 504 describes an assay for detecting hepatitis A antigen in a sample involving contacting a surface having hepatitis A antibody adsorbed thereto with a proteinaceous material capable of being absorbed on the surface. Such surface is then contacted with a sample containing hepatitis A antigen. The surface is thereafter incubated to form a complex of hepatitis A antigen and hepatitis A antibody. In other words, the antigen and the antibody are not bound separately on the substrate. Furthermore, there is no disclosure of processes for the detection of antibodies.

EP—A—0 019 217 describes a process for the detection of antigens in a test specimen comprising the steps of

A) contacting a test specimen suspected of containing a given antigen with a substrate coated with antibodies of said antigen, incubating the contacted substrate and washing the substrate;

B) contacting the washed material of step A) with a hapten conjugated antibody against said antigen, incubating the so-called material and washing the so-incubated material;

C) contacting the washed material of step B) with a radioactive material labeled or enzyme labeled anti-hapten antibody, incubating the so-contacted material and washing the same; and

D) effecting radioimmunoassay if said antibody is radioactive or enzyme labeled immunoassay if said antibody contains an enzyme moiety.

By conducting the process thusly, antigen can be determined without employing a purified source of antigen to prepare purified antibody. Detection of the antigen content in the specimen is effected by comparing the counts derived from the radioimmunoassay or the enzyme concentration in the case of enzyme labeled immunoassay (ELISA) with a standard known to be free of the antigen. Quantitative determination is effected by comparing the counts or enzyme concentration against data derived from the same test protocol using samples of known antigen concentrations.

The present invention takes advantage of the ability of anti-hapten antibody to readily react with hapten groups on the antibody employed in step B) which has, in turn, reacted with antigen present in the test specimen. This antigen present in the test specimen has previously react with the corresponding antibody held on the substrate. By this technique, the initial antibody employed need not be particularly pure and the quantity of antigen in the specimen is readily detected owing to magnification of test results as a result of the described sandwich technique wherein hapten conjugated groups on the antibody are reacted with anti-hapten antibody. In accordance with that process, the substrate containing the antibody is contacted with test specimens containing the suspected antigen. The suspected antigen reacts with the antibody on the substrate and, in turn, is available for further reaction with the hapten conjugated antibody. When in accordance with step "B)" of such process, the hapten conjugated antibody contacts the antibody—antigen product resulting from step "A)", there is formed a sandwich structure wherein the antigen is sandwiched on the one side by the substrate—antibody reagent and on the other side by the hapten conjugated antibody.

The sandwich structure which results, has available hapten groups, since it is the antibody portion of the hapten conjugated antibody that reacts with the antigen held by the substrate—antibody material used in step "A)". This makes the hapten groups on the hapten conjugated antibody readily available for reaction with radio- or enzyme labeled anti-hapten antibody. Since the hapten conjugated antibody contains multiple hapten moieties, subsequent reaction with the labeled anti-hapten anti-bodies provides a substance which provides a magnified count whether analysis be by radioimmunoassay or ELISA. In other words, since the quantity of hapten moieties on the hapten conjugated antibody is higher than the number of antigens adsorbed, a greater number of anti-hapten antibodies will react with those sites.

This means that the number of counts per antigen is greater than in the standard radioimmunoassay techniques. This magnification permits the measurement of nanogram quantities of antigen in the test specimen. It is this magnification be the use of hapten conjugated antibody that permits use of antibody reagents in step "A)" which are not particularly pure.

EP—A— 0 080 109 discloses a process for determining the presence of an antigen or antibody in a sample wherein such antigen or antibody exists in the form of an immune complex which process comprises:

A) contacting the immune complex originating from the sample with a dissociating buffer whereby said immune complex, if present, is dissociated into antigen and antibody;

B) contacting a solid support which binds proteins with said dissociating buffer suspected of containing antigen or antibody and removing said buffer;

C) washing said solid support;

D) adding protein to fill unoccupied sites on said solid support;

E) adding radioactively labeled or enzyme labeled antibody or antigen to said solid support, incubating the resultant mass and washing the same;

F) measuring the radioactivity or enzymatic activity associated with the solid support;

Said process can be performed by isolation of the immune complex from other components with which it is in admixture, normally other proteins. This isolation can involve the removal of immune complexes from sera in which they are present by methods generally known in the art. Accordingly, the immune complexes can be adsorbed onto a solid support or otherwise precipitated from the serum containing the same. This can be effected by precipitation with polyethylene glycol and subsequent adsorption of the immune complex on material such as staphylococci-carrying protein A, by the use of protein A linked to a solid support such as agarose. Alternatively, one can use conglutinin linked to a solid support as adsorbent for the immune complex. After effecting adsorption of the immune complex onto a solid support the serum is removed therefrom, and the solid support is washed to remove excess proteins. Thereafter, whether the antigen and antibody is adsorbed to the solid support, the same is brought in contact with a dissociating buffer which dissociates the immune complex. If the immune complex is one which has been adsorbed to the solid support, not only is the immune complex dissociated into the antibody and antigen components but it is also removed from the solid support. To achieve this, dissociating buffers are employed such as urea, guanidine hydrochloride, thiocyanate salts such as sodium and potassium thiocyanate, magnesium chloride, lithium diiodosalicylate or solutions of low (2—3.5) or high (10—11.5) pH. It is important that the buffer be one which substantially completely dissociates the immune complex from the solid support and dissociates the immune complex itself into its component antigen and antibody.

Dissociation of the immune complex is generally carried out at a temperature between 0 and 45°C, preferably room temperature, the dissociating buffer being brought into contact with the immune complex adsorbed on to the solid support for at least two minutes, preferably at least five minutes, and generally between five and 30 minutes. Thereafter, the solid support originally bearing the immune complex is separated from the dissociating buffer which is now suspected of containing antigen or antibody (derived from the immune complex contained in the original sample).

The dissociating buffer suspected of containing antigen or antibody is thereafter brought in contact with the solid support which binds proteins. For this purpose, a wide variety of supports can be used. For instance, one can employ a paper type material which has been treated with a protein binding agent such as diazobenzyloxymethyl paper or diazophenylthioether paper. Treatment of paper with these agents renders the paper capable of binding proteins. Alternatively, one can use a nitrocellulose sheet or similar material. In particular, it is contemplated to use as the solid support a plastic material such as a polystyrene, polyvinyl e.g. polyvinylchloride, polyvinylidene chloride, polyacrylonitrile, polyvinylacetate. Other plastics which can be used include polyethylene, polypropylene, nylon, and derivatized glass.

Quite surprisingly, when these and other protein-adsorbing solid supports are brought in contact with dissociating buffers suspected of containing the antigen or antibody derived from the immune complex, irreversible attachment of the proteins is effected, notwithstanding the copresence of those components in the dissociating buffer which dissociated the antigen antibody and served to remove the antibody from the solid support. In particular, it was quite surprisingly observed that antigen or antibodies in the dissociating buffer attached to a wide variety of solid matrixes and in such environment the presence of the dissociating buffer does not minimize the attachment of the protein to the solid matrixes nor does it cause elution of proteins which had been adsorbed to such matrixes. By effecting adsorption of antigen or antibody derived from the immune complex on the solid support, the existence of antibody of antigen can be determined by a simple radioimmunoassay or enzyme labeled immunoassay technique.

It was now discovered that the product resulting from contact of such solid support which binds proteins with dissociating buffer containing dissociated antigen and antibody can itself be used for various antigen and/or antibody detection and specifically for the purpose of EP—A—0 019 277 to bind an antibody or antibody in a test specimen as the antibodies and antigens present on the solid support which binds proteins are each bound directly to the solid support and do not exist in the form of an immune complex. Thus, both the antigens and antibodies are each available to detect the presence of a corresponding antibody or antigen in a specimen.

Broadly, the present invention contemplates a diagnostic reagent as defined supra which is characterized in that the substrate has bound thereon a mixture of the antigen and of the antibody originating from a dissociated immune complex, said antibodies and said antigens bound to said substrate being separately bound to said substrate and not in the form of an immune complex.

The invention further contemplates the use of the diagnostic reagent as defined above for the detection of an antigen in a specimen, comprising

a) contacting said specimen with said substrate, incubating the so-contacted substrate and washing the substrate;

b) contacting the washed material of step 'a' with a radioactively labeled or enzyme labeled antibody, incubating the so-contacted material and washing the same; and

c) effecting radioimmunoassay if said antibody is radioactive or enzyme labeled immunoassay if said antibody is enzyme labeled.

The present invention also contemplates the use of the diagnostic reagent defined above, for the detection of an antibody in a test specimen, comprising

a) contacting said specimen suspected of containing antibody with said substrate, incubating the so-contacted substrate and washed the substrate;

b) contacting the washed material of step "a" with a radioactively labeled or enzyme labeled antigen, incubating the same and washing the same; and

c) effecting radioimmunoassay if said antigen is radioactive or enzyme labeled immunoassay if said antigen is enzyme labeled.

The present invention can also be employed to detect antigens in specimens by competition test. The use of the diagnostic reagent for such purpose comprises:

a) contacting a specimen suspected of containing an antigen with said substrate in the presence of a radioactively labeled or enzyme labeled antigen, incubating the so-contacted substrate and washing the substrate;

b) effecting radioimmunoassay if said antigen is radioactive or enzyme labeled immunoassay if said antigen is enzyme labeled; and

c) comparing the counts if radioimmunoassay is effected or enzymatic activity is enzyme labeled immunoassay is effected with the counts or enzymatic activity of a control carried out without the presence of the specimen. In such case, the presence of antigen is indicated by decreased counts if radioimmunoassay is effected or decreased enzyme activity if enzyme labeled immunoassay is effected.

The present invention can further be employed to determine the presence of antibodies in specimens by competition tests according to which the use of the diagnostic reagent comprises:

a) contacting said specimen suspected of containing antibody with said substrate, in the presence of a radioactively labeled or enzyme labeled antibody, incubating the so-contacted substrate and washing the substrate; and

b) comparing the counts or enzymatic activity with the counts or enzymatic activity of a control carried out without the use of a specimen. The presence of antibody is indicated by decreased counts or decreased counts or decreased enzyme activity.

In a specific embodiment, the diagnostic reagent of the present invention, when applied to the detection of antigen, is used such that the washed material of step "a" is contacted with a hapten conjugated antibody against said antigen of said specimen, the so-contacted material is incubated and washed, and thereafter, the resultant washed material is contacted with a radioactively labeled or enzyme anti-hapten antibody, incubated and washed. and radioimmunoassay is effected if said anti-hapten antibody is radiolabeled or enzyme labeled immunoassay is effected if said anti-hapten antibody is enzyme labeled.

When applied to the detection of the presence of an antibody in a specimen, a specific embodiment or using the diagnostic reagent of the present invention is characterized in that, following step 'a', the washed material of step 'a' is contacted with a hapten conjugated antigen against said antibody, the so-contacted material is incubated and washed and the resultant washed material is contacted with as radioactively labeled or enzyme containing anti-hapten-antibody, incubated and washed, after which radioimmunoassay is effected if said antibody is radioactive or enzyme labeled immunoassay is effected if said antibody is enzyme labeled.

The heart of the present invention lies in the use of a novel material obtained during the immunoassay according to EP—A—0 080 109. That material comprises the solid support which binds protein onto which there is separately bound a mixture of antigens and antibodies where the antigens are directly bound to the solid support and the antibodies themselves are directly bound to the solid support and the antigens and antibodies so-bound to the solid support do not exist in the form or an immune complex.

This imtermediate is therefore useful as the substrate in the process of EP—A—0 019 277, discussed supra.

It is to be understood that the heart of the present invention lies in the fact that one can convert an immune complex into a useful diagnostic aid by dissociating that immune complex in a dissociating buffer, whether or not the immune complex has been pre-disposed on a solid support such as Staphylococcoi protein A or the like. That dissociating buffer containing dissociated antigen and antibody can form a valuable diagnostic aid simply by contacting that mixture with a solid support which binds proteins. Following incubation and washing and separation of the so-contacted protein binding support, there is obtained a useful diagnostic agent. Of course, one need not obtain the protein binding solid support—antibody, antigen material as a result of another assay. The heart of the invention resides in the conversion of an immune complex into its component antigen and antibody and the disposition of antigens and antibodies separately from one another, and not in a form of an immune complex, onto a protein binding solid support. Once this is obtained, the resultant reagent can be used in the detection of antigens or antibodies by the procedures described above. Of course, when used in the detection of an antigen, for instance, the antigen which is copresent on the protein-binding support has no special function since the antibody also present thereon is that which binds the antigen of the specimen. The reagent, however, is also useful in the separate detection of antibodies in a specimen where the specimen suspected of containing antibody is brought in contact with the substrate having bound thereon a mixture of antibody and antigen, in which case the antibody bound to the substrate performs no function. Nevertheless, from one treatment there is obtained a diagnostic reagent which is useful in a number of different assays.

Generally speaking, the protein binding solid support is treated with the dissociating buffer containing antigen and antibody at a temperature between 0 and 45°, preferably between 15 and 25°C for at least 60 minutes, preferably at least two hours and generally overnight. This ensures maximum adsorption of antigen and antibody, separately from one another, onto the protein binding solid support. Is should be noted that for this adsorption it is unnecessary that the solid protein binding sorbent be pretreated with a portein binding agent. After the protein solid sorbent is treated with the dissociating buffer containing antigen and antibody, the solid support is washed free of extraneous material.

Thereafter, those sites on the solid support not occupied by antigen or antibody are filled with protein so that substantially all of the available sites on the protein binding solid support are occupied. This ensures that upon subsequent addition of a test specimen containing antigen or antibody as the case may be, reaction occurs predominantly or only upon the pre-deposited antigen or antibody derived from the dissociating buffer containing composition.

To this end, one can use a wide variety of materials to occupy the unoccupied binding sites on the solid support. These include in particular proteins, notably the following binding site occupiers: bovine serum albumin, gelatine, normal human serum or animal sera (bovine, calf, fetal calf, chicken, etc.). Preferably the solid support is incubated with a solution of bovine serum albumin to saturate the unoccupied protein binding sites.

Thereafter, the sample is washed free of extraneous material, and there is added the test specimens suspected of containing antigen or antibody as the case may be.

The process of the invention can be used with respect to any antigen, the presence of which is suspected in a given serum. All that is required is that an antibody to such suspected antigen be deposited on a substrate, that the specimen containing the suspected antigen contact the antibody on the substrate, incubation is effected and the so-incubated material is washed. Thereafter, in accordance with the second procedural series of steps, the washed material is contacted with hapten conjugated antibody against said antigen, which contacting is also followed by incubation and washing. These steps provide the hapten moieties on the antibody against the suspected antigen, which hapten moieties will react with radioactive labeled or enzyme containing anti-hapten antibody. Thereafter, the anti-hapten antibody which is either radioactive labeled or contains an enzyme is contacted with the washed material which is followed by incubation and washing. Radioimmunoassay or enzyme labeled immunoassay is effect to determine qualitatively the presence of the antigen and quantitatively the amount of antigen by comparison with prepared standards. The higher the counts from a γ-counter or higher concentration of enzyme, the higher is the quantity of antigen in the test specimen.

Antigen whose presence and amount can be detected in accordance with the claimed process include essentially *any* antigen, for example viral, e.g., hepatitis B, influenza, adenovirus, and all other viral antigens, as well as bacterial antigens, tumor-specific antigens, serum antigens, enzyme proteins and all other antigens having at least two antigenic sites.

The antibodies of these antigens can be hapten conjugated with a wide variety of haptens including those which provide the following hapten moieties: dinitrophenyl, trinitrophenyl, diazotized sulfanilic acid, p-azobenzene arsonate, benzyl penicillin, p-azobenzoate, aspirin, fluorescein isothiocyanate, p-iodobenzoate, p-(p'-hydroxyphenylazo) benzoate, phosphorylcholine and others.

The conjugation of haptens with proteins and the preparation of anti-hapten antibodies and their properties have been extensively reviewed (see, for example: "Advanced Immunochemistry", E. D. Day, Williams E. Williams, Baltimore, 1972; A. L. deWeck, "Low Molecular Weight Antigens" in: THE ANTIGENS, Ed. M. Sela, Academic Press, New York, 1974, volume 2, pages 142—249).

Anti-hapten antibodies can be formed which correspond, in respect of the hapten moiety, to the hapten moiety on the conjugated antibody. Thus, the labeled anti-hapten antibody used in step "C)" corresponds with respect to its hapten moiety to the hapten moiety of the hapten conjugated antibody against the suspected antigen. The same can be prepared in known manner, as by haptenating an antigen and introducing the so-haptenated antigen into a test animal, such as a rabbit, to effect an antibody response. As a result thereof, as is known, there is formed the antibody to the antigen and an anti-hapten antibody. The resultant serum is recovered and the anti-hapten antibody is separated from the other serum proteins, including the antibody to the original antigen.

The anti-hapten antibody is thereafter labeled, either with a radioactive material such as $I^{125}$ or $I^{131}$ or is conjugated with an enzyme whereby there is formed an enzyme-containing anti-hapten antibody. This enzyme-containing anti-hapten antibody can then be used as a "labeled" anti-hapten antibody-labeled in the sense that it contains an enzyme, but is not radioactive. Detection of the adsorption of the "labeled" anti-hapten antibody can be by RIA or ELISA in accordance with known techniques. RIA involves the use of a radiation detection means, whereas ELISA involves a measurement of the concentration of enzyme. The higher the enzyme concentration, the higher is the concentration of antigen adsorbed and the concentration of antigen in the original test specimen.

The incubation required in accordance with steps A), B), and C) can be effected in known manner, such as under the following conditions: 1—8 hours at 37—50°C and 16—72 hours at 18—30°C.

Washing is typically effected using an aqueous solution such as one buffered at a pH of 6—8, preferably at a pH of about 7, employing an isotonic saline solution.

The process of the invention is, of course, equally applicable to the detection of the presence of an antibody in a specimen by an analogous procedure wherein the specimen suspected of containing an antibody is brought in contact with the reagent comprising a mixture of antibodies and antigens on a protein-binding substrate followed by incubation and washing. In such instance the so-washed material is thereafter contacted with a hapten-conjugated antigen, incubated and washed. Thereafter, the material so-obtained is brought in contact with the radioactive material labeled or enzyme containing anti-hapten antibody. Following incubation and washing, radio immunoassay or enzyme labeled immunoassay is effected depending upon whether or not a radioactive or enzyme-containing anti-hapten antibody was employed.

In carrying out the process of the invention, it is preferred that in step "C)" there be used radioactive material labeled or enzyme containing anti-hapten antibodies which have been obtained by dissociation of an anti-hapten antibody-antigen immune complex. Such anti-hapten antibodies can be prepared by haptenating an antigen and introducing the so-haptenated antigen into test animal such as a rabbit to effect an antibody response. As a result thereof, there is obtained an immune complex which can be dissociated by use or a dissociating buffer such as one of those listed above and the anti-hapten antibody can be recovered by column chromatography. For instance, anti-DNP can be isolated from anti-DNPBSA (bovine serum albumin) by contacting 2 ml of the anti-serum with 10 mg DNP-apoferritin followed by incubation for one hour at 37°C, overnight at 4°C, and centrifugation for one hour at 90,000 xg. The resultant pellet is dissolved in 1 ml of 8 molar urea—0.01 molar phosphate pH 8.0—0.1% Nonidet P-40® (BDH Chemicals, Ltd., Poole, England) (UPN) and applied to a 2 ml column of DEAE-cellulose prewashed with UPN. Anti-DNP IgG can be recovered from the void volume of the column following elution with UPN.

In order to more fully illustrate the nature of the invention and the manner of practising the same, the following is presented.

Example
Development of a test for Antibodies to Hepatitis B Core Antigen [ANTI-HB$_c$]

A) Preparation of ($^{125}$I) labeled Anti-HB$_c$.

Anti-HB$_c$ was isolated from anti-HB$_c$-positive sera by chromatography on DEAE-cellulose (Fudenberg, H. H. (1967) in Methods in Immunology and Immunochemisty, eds. Williams, C. A. and Chase, M. W. (Academic Press, New York), Vol. 1, pp 307—385) and labeled with $^{125}$I by one of the standard radiolabeling techniques.

B) Preparation of Polystyrene Beads Created with HB$_c$Ag originating from an Immune Complex.

Serum containing HBV (hepatitis B virus) was treated for 1 hour at 37°C with 2-mercaptoethanol (100 µg/ml) and the detergent Nonidet P-40 (1 mg/ml) to release HB$_c$Ag from HBV particles. Since practically all sera containing HBV also contain an excess of anti-HB$_c$, HB$_c$Ag-anti-HB$_c$ complexes are formed. These complexes were isolated in the following way.

One part of treated serum was mixed with 9 perts of 0.14 M NaCl—0.01 M Tris pH 7.2 (TS), incubated at 37°C for 45 min. and centrifuged for 20 min. at 1,700 × g without refrigeration. The supernatant fluids were mixed with an equal volume of ice-cold TS containing 40 g/liter of PEG and 30 g/liter of Tween 20®. After standing 90 min. at 0°C. the samples were centrifiged for 20 min. at 1,700 × g(4°C). The precipitates were washed twice with 30 parts each of TS containing 25 g/liter of PEG and 15 g/liter of Tween 20. The final pellets after recentrifugation contained 0.1 to 0.2% of protein originally present in serum. The isolated immune complexes were dissolved in 3 M NaSCN and used for coating of polystyrene beads. After overnight incubation at 20°C, the beads were used for tests.

C) Performance of Tests

Specimens (400 µl of anti-HB$_c$-positive sera and negative controls, respectively, were added to the beads together with a constant amount of $^{125}$I-anti-HB$_c$. The beads were incubated at room temperature, preferably overnight, washed with Tris-buffered saline and counted in a γ counter.

Test results:
  negative conrol = A cpm
  positive control = B cpm
where A > B (since unlabeled anti-HB$_c$ competes with $^{125}$I-anti-HB$_c$ for the same sites on HB$_c$A$_g$-coated beads)
  All specimens for which cpm ≤

$$\frac{A}{2}$$

are considered positive. For titration of anti-HB$_c$ in a specimen, the latter is serially diluted in normal human serum and tested. The highest dilution for which cpm ≤

$$\frac{A}{2}$$

corresponds to the endpoint titer.

**Claims**

1. A diagnostic reagent which is suitable both for the detection of an antigen, and for the detection of an antibody in a specimen, by radioimmunoassay or by enzyme labeled immunoassay, said reagent comprising a substrate having bound thereon an antibody and an antigen, characterized in that said substrate has bound thereon a mixture of said antigen and of said antibody originating from a dissociated immune complex, said antibodies and said antigens bound to said substrate being separately bound to said substrate and not in the form of an immune complex.

2. A reagent according to claim 1, characterized in that said substrate is a protein binding solid support in which the sites free of antigen and antibody are substantially filled with a protein binding site occupier.

3. A reagent according to claim 2, characterized in that said protein site occupier is bovine serum albumin, gelatin, a normal human serum or an animal serum.

4. A reagent according to claim 2 or 3, characterized in that said protein binding solid support is derivatized paper, such as nitrocellulose paper, a diazobenzyloxymethyl paper or a diazophenylthioether paper; or is or comprises a plastic material, such as polystyrene, a polyvinyl material, or a polyolefin.

5. The use of the diagnostic reagent according to any one of claims 1 to 4, for the detection of an antigen in a specimen, comprising
   a) contacting said specimen with said substrate, incubating the so-contacted substrate and washing the substrate;
   b) contacting the washed material of step 'a' with a radioactively labeled or enzyme labeled antibody, incubating the so-contracted material and washing the same; and
   c) effecting radioimmunoassay if said antibody is radioactive or enzyme labeled immumoassay if said antibody is enzyme labeled.

6. The use according to claim 5, characterized in that the washed material of step 'a' is contacted with a hapten conjugated antibody against said antigen of said specimen, the so-contacted material is incubated and washed and thereafter the resultant washed material is contacted with a radioactively labeled or enzyme labeled anti-hapten antibody, incubated and washed; and radioimmunoassay is effected if said anti-hapten antibody is radio labeled or enzyme labeled immunoassay is effected if said anti-hapten antibody is enzyme labeled.

7. The use of the diagnostic reagent according to any one of claims 1 to 4 for detecting the presence of a antigen in a specimen, comprising
   a) contacting the specimen suspected of containing an antigen with said substrate, in the presence of a radioactively labeled or enzyme labeled antigen, incubating the so-contacted substrate and washing the substrate;
   b) effecting radioimmunoassay if said antigen is radioactive or enzyme labeled immunoassay if said antigen is enzyme labeled; and
   c) comparing the counts if radioimmunoassay is effected or enzymatic activity if enzyme immunoassay is effected with the counts or enzymatic activity of a control carried out without the presence of said specimen.

8. The use of the diagnostic reagent according to any one of claims 1 to 4 for the detection of the presence of an antibody in a test specimen, comprising
   a) contacting said specimen suspected of containing antibody with said substrate, incubating the so-contacted substrate and washing the substrate;
   b) contacting the washed material of step 'a' with a radioactively labeled or enzyme labeled antigen, incubating the same and washing the same; and
   c) effecting radioimmunoassay if said antigen is radioactive or enzyme labeled immunoassay if said antigen is enzyme labeled.

9. The use according to claim 8. characterized in that following step 'a', the washed material of step 'a' is contacted with a hapten conjugated antigen against said antibody, the so-contacted material is incubated and washed and the resultant washed material is contacted with a radioactively labeled or enzyme labeled anti-hapten antibody incubated and washed after which radioimmunoassay is effected if said antibody is radio active or enzyme labeled immunoassay is effected if said antibody is enzyme labeled.

10. The use of the diagnostic reagent according to any one of claims 1 to 4 for the detection of the presence of an antibody in a specimen, comprising
   a) contacting said specimen suspected of containing said antibody with said substrate, in the presence of a radioactively labeled or enzyme antibody, incubating the so-contacted substrate and washing the substrate; and
   b) comparing the counts or enzymatic activity with the counts or enzymatic activity of a control carrier without the use of said specimen.

**Patentansprüche**

1. Diagnostisches Reagens, das sowohl für den Nachweis eines Antigens, als auch für den Nachweis eines Antikörpers in einer Probe durch Radioimmunoassay oder durch Enzymmarkierungs-Immunoassay geeignet ist, wobei das Reagens ein Substrat enthält, an welchem ein Antikörper und ein Antigen gebunden sind, dadurch gekennzeichnet, daß an dem Substrat ein Gemisch des Antigens und des Antikörpers, das aus einem dissoziierten Immunkomplex stammt, gebunden ist, wobei die an das Substrat gebundenen Antikörper und Antigene getrennt und nicht in Form eines Immunkomplexes an das Substrat gebunden sind.

2. Reagens nach Anspruch 1, dadurch gekennzeichnet, daß das Substrat einenfesten, proteinbindenden Träger darstellt, bei welchem die von Antigen oder Antikörper frei Stellen in wesentlichen mit einem die Bidungsstellen besetzenden Protein aufgefüllt sind.

3. Reagens nach Anspruch 2, dadurch gekennzeichnet, daß das die Bindungsstellen besetzende Protein Rinderserumalbumin, Gelatine, ein normales menschliches Serum oder ein tierisches Serum darstellt.

4. Reagens nach Anspruch 2 oder 3, dadurch gekennzeichnet daß der das Protein bindende feste Träger derivatisiertes Papier, z.B. Nitrocellulosepapier, ein Diazobenzyloxymethylpapier oder ein Diazophenylthioätherpapier darstellt bzw. ein Kunststoffmaterial, wie Polystyrol, ein Polyvinylmaterial oder ein Polyolefin darstellt oder enthält.

5. Verwendung des diagnostischen Reagens nach einem der Ansprüche 1 bis 4 zum Nachweis eines Antigens in einer Probe, wobei.

a) die Probe mit dem Substrat kontaktiert wird, das so kontaktierte Substrat inkubiert und das Substrat gewaschen wird;

b) das gewaschene Material von Stufe "a" mit einem Antikörper, der mit einem radioaktiven Material oder einem Enzym markiert ist, kontaktiert wird, das so kontaktierte Material inkubiert und gewashcen wird; und

c) ein Radioimmunoassay durchgeführt wird, wenn der Antikörper radioaktiv ist, oder ein Enzymmarkierungs-Immunoassay durchgeführt wird, wenn der Antikörper enzymmarkiert ist.

6. Verwendung nach Anspruch 5, dadurch gekennzeichnet, daß das gewaschene Material von Stufe "a" mit einem Antikörper gegen das Antigen der Probe, der mit einem Hapten konjugiert ist, kontaktiert wird, das so kontaktierte Material inkubiert und gewaschen wird und das erhaltene gewaschene Material anschließend mit einem Anti-Hapten-Antikörper, der mit einem radioaktiven Material oder mit einem Enzym markiert ist, kontaktiert, inkubiert und gewaschen wird, und daß ein Radioimmunoassay durchgeführt wird, wenn der Anti-Hapten Antikörper radioaktiv markiert ist oder ein Enzymmarkierungs-Immunoassay durchgeführt wird, wenn der Anti-Hapten-Antikörper enzymmarkiert ist.

7. Verwendung des diagnostischen Reagens nach einem der Ansprüche 1 bis 4 zum Nachweis eines Antigens in einer Probe, wobei

a) die Probe, von der vermutet wird, daß sie ein Antigen enthält, mit dem Substrat in Gegenwart eines Antigens, das mit einem radioaktiven Material oder mit einem Enzym markiert ist, kontaktiert wird, das so kontaktierte Substrat inkubiert wird und das Substrat gewaschen wird;

b) ein Radioimmunoassay durchgeführt wird, wenn das Antigen radioaktiv markiert ist oder ein Enzymmarkierungs-Immunoassay durchgeführt wird, wenn das Antigen enzymmarkiert ist; und

c) wobei die Impulse (bei Durchführung eines Radioimmunoassay) bzw. die enzymatische Aktivität (bei Durchführung eines Enzym-Immunoassay) mit den Impulsen bzw. der enzymatischen Aktivität eines Kontrolle, die in Abwesenheit der Probe druchgeführt wurde, verglichen werden.

8. Verwendung des diagnostischen Reagens nach einem der Ansprüche 1 bis 4 zum Nachweis eines Antikörpers in einer Testprobe, wobei

a) die Probe, von der vermutet wird, daß sie den Antikörper enthält mit dem Substrat kontaktiert wird, das so kontaktierte Substrat inkubiert und das Substrat gewaschen wird;

b) das gewaschene Material von Stufe "a" mit einem Antigen, das mit einem radioaktiven Material oder einem Enzym markiert ist, kontaktiert wird, dasselbe inkubiert und gewaschen wird; und

c) ein Radioimmunoassay durchgeführt wird, wenn das Antigen radioaktiv ist oder ein Enzymmarkierungs-Immunoassay durchgeführt wird, wenn das Antigen enzymmarkiert ist.

9. Verwendung nach Anspruch 8, dadurch gekennzeichnet, daß nach der Stufe "a" das gewaschene Material von Stufe "a" mit einem Antigen gegen den Antikörper, das mit einem Hapten konjugiert ist, kontaktiert wird, das so kontaktierte Material inkubiert und gewaschen und das erhaltene gewaschene Material mit einem Anti-Hapten-Antikörper, der mit einem radioaktiven Material markiert ist oder ein Enzym enthält, kontaktiert, inkubiert und gewaschen wird, worauf ein Radioimmunoassay durchgeführt wird, wenn der Antikörper radioaktiv ist oder ein Enzymmarkierungs-Immunoassay durchgeführt wird, wenn der Antikörper enzymmarkiert ist.

10. Verwendung des diagnostischen Reagens nach einem der Ansprüche 1 bis 4 zum Nachweis eines Antikörpers in einer Probe, wobei

a) die Probe, von der vermutet wird, daß sie den Antikörper enthält, mit dem Substrat in Gegenwart eines Antikörpers, der mit einem radioaktiven Material oder mit einem Enzymmarkiert ist, kontaktiert wird, das so kontaktierte Substrat inkubiert und das Substrat gewaschen wird; und

b) die Impulse oder die enzymatische Aktivität mit den Impulsen oder der enzymatischen Aktivität eines Kontrollträgers ohne Verwendung der Probe verglichen werden.

## Revendications

1. Réactif diagnostique pourvant être utilisé tant pour la détection d'un antigène que pour la détection d'un anticorps dans un échantillon, par analyse radioimmunologique ou analyse immunologique à marquage enzymatique, ledit réactif comprenant un substrat sur lequel sont fixés un anticorps et un antigène, caractérisé en ce que sur le substrat est fixé un mélange dudit antigène et dudit anti-corps provenant d'un complexe immun dissocié, lesdits anticorps et lesdits antigènes fixés audit substrat étant séparément fixés audit substrat et non sous la forme d'un complexe immun.

2. Réactif selon la revendication 1, caractérisé en ce que ledit substrat est un support solide fixant les protéines, dans lequel les sites exempts d'antigène et d'anticorps sont pratiquement remplis d'un occupant de site de fixation des protéines.

3. Réactif selon la revendication 2, caractérisé en ce que ledit occupant du site de fixation des protéines est l'albumine de sérum de boeuf, la gélatine, un sérum humain normal ou un sérum animal.

4. Réactif selon la revendication 2 ou 3, caractérisé en ce que ledit support solide de fixation des protéines est un dérivé du papier, comme un papier de nitrocellulose, un papier de diazobenzyloxyméthyle ou un papier de diazophénylthioéther; ou bien est ou comprend une matière plastique, comme un polystyrène, une matière polyvinylique ou une polyoléfine.

5. Utilisation du réactif diagnostique selon l'une quelconque des revendications 1 à 4 pour la détection d'un antigène dans un échantillon, consistant

a) à mettre en contact ledit échantillon avec ledit substrat, à incuber le substrat ainsi mis en contact et à laver le substrat;

b) à mettre en contact la matière lavée de l'étape "a" avec un anticorps radiomarqué ou marqué par une enzyme, à incuber la matière ainsi mise en contact et a la laver; et

c) à effectuer une analyse radioimmulologique si ledit anticorps est radioactif, ou une analyse enzymoimmunologique si ledit anticorps est marqué par une enzyme.

6. Utilisation selon la revendication 5, caractérisée en ce que la matière lavée de l'étape "a" est mise en contact avec un anticorps conjugué d'un haptène contre ledit antigène dudit échantillon, la matière ainsi mise en contact est incubée et lavée, puis la matière lavée obtenue est mise en contact avec un anticorps anti-haptène radiomarqué ou marqué par une enzyme, incubée et lavée, et on effectue une analyse radioimmunologique si ledit anticorps anti-haptène est radiomarqué, ou une analyse enzymoimmunologique si ledit anticorps anti-haptène est marqué par une enzyme.

7. Utilisation du réactif diagnostic selon l'une quelconque des revendications 1 à 4 pour détecter la présence d'un antigène dans un échantillon, consistant

a) à mettre en contact l'échantillon suspecté de contenir un antigène avec ledit substrat, en présence d'un antigène radiomarqué ou marqué par une enzyme, à incuber le substrat ainsi mis en contact et à laver le substrat;

b) à effectuer une analyse radioimmunologique si ledit antigène est radiomarqué ou une analyse enzymo-immunologique si ledit antigène est marqué par une enzyme; et

c) à comparer les nombres de coups si l'on effectue une analyse radioimmunologique ou les activités enzymatiques si l'on effectue une analyse enzymoimmunologique, avec les coups par minute ou l'activité enzymatique d'un témoin réalisé en l'absence dudit échantillon.

8. Utilisation du réactif diagnostic selon l'une quelconque des revendications 1 à 4 pour la détection de la présence d'un anticorps dans l'échantillon d'essai, consistant

a) à mettre en contact avec ledit substrat ledit échantillon suspecté de contenir un anticorps, à incuber le substrat ainsi contacté et à laver le substrat;

b) à mettre en contact la matière lavée de l'étape "a" avec un antigène radiomarqué ou marqué par une enzyme, à l'incuber et à la laver; et

c) à effectuer une analyse radioimmunologique si ledit antigène est radiomarqué ou une analyse enzymoimmunoligique si ledit antigène est marqué par une enzyme.

9. Utilisation selon la revendication 8, caractérisée en ce que, après l'étape "a", la matière lavée de l'étape "a" est mise en contact avec un antigène conjugué de l'haptène contre ledit anticorps, la matière ainsi en contact est incubée et lavée, et la matière lavée obtenue est mise en contact avec un anticorps anti-haptène radiomarqué ou marqué par une enzyme incubée et lavée, ce après quoi on effectue une analyse radioimmunologique si ledit anticorps est radiomarqué ou une analyse enzymoimmunologique si ledit anticorps est marqué par une enzyme.

10. Utilisation du réactif diagnostic selon l'une quelconque des revendications 1 à 4 pour la détection de la présence d'un anticorps dans un échantillon, consistant

a) à mettre en contact avec ledit substrat ledit échantillon suspecté de contenir ledit anticorps, en présence d'un anticorps radiomarqué ou marqué par une enzyme, à incuber le substrat ainsi mis en contact et à laver le substrat; et

b) à comparer les coups par minute ou l'activité enzymatique avec les coups par minute ou l'activité enzymatique d'un support témoin sans utiliser ledit échantillon.